(19) Europäisches Patentamt
European Patent Office
Office européen des brevets

(11) **EP 4 570 373 A1**

(12) **EUROPEAN PATENT APPLICATION**
published in accordance with Art. 153(4) EPC

(43) Date of publication:
**18.06.2025 Bulletin 2025/25**

(21) Application number: 23866983.2

(22) Date of filing: 07.06.2023

(51) International Patent Classification (IPC):
***B01J 21/18*** (2006.01)   ***B01J 23/72*** (2006.01)
***B01J 37/00*** (2006.01)

(52) Cooperative Patent Classification (CPC):
**B01J 20/06; B01J 20/20; B01J 20/30; B01J 21/18;
B01J 23/72; B01J 35/30; B01J 35/33; B01J 35/39;
B01J 35/45; B01J 35/61; B01J 37/00; C01B 32/40;
C07C 29/154; C10G 2/00; C25B 1/23;** (Cont.)

(86) International application number:
**PCT/CN2023/098776**

(87) International publication number:
**WO 2024/060687 (28.03.2024 Gazette 2024/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 20.09.2022 CN 202211146205

(71) Applicants:
• **China Petroleum & Chemical Corporation
Beijing 100728 (CN)**
• **Sinopec Dalian Research Institute of
Petroleum and Petrochemicals Co., Ltd.
Lushunkou District
Dalian, Liaoning 116045 (CN)**

(72) Inventors:
• **CAI, Haile**
**Dalian, Liaoning 116045 (CN)**
• **ZHANG, Shudong**
**Dalian, Liaoning 116045 (CN)**
• **SONG, Yongyi**
**Dalian, Liaoning 116045 (CN)**
• **MA, Rui**
**Dalian, Liaoning 116045 (CN)**
• **ZHAO, Liping**
**Dalian, Liaoning 116045 (CN)**

(74) Representative: **karo IP
Patentanwälte PartG mbB
Steinstraße 16-18
40212 Düsseldorf (DE)**

(54) **COPPER-CARBON COMPOSITE MATERIAL, PREPARATION METHOD THEREFOR AND USE THEREOF**

(57) The present application relates to a copper-carbon composite material, its preparation and application thereof. The copper-carbon composite material has the advantages of high catalytic activity and high stability. The copper-carbon composite material comprises an active component and a carrier, wherein the active component comprises a combination of Cu and $Cu_2O$, the carrier is a porous carbonaceous material, the content of the combination is 1 wt% to 50wt%, calculated based on the mass of Cu element, relative to the composite material as 100 wt%, and the R1 value of the composite material is 0.4-2 : 1.

Fig. 1

(52) Cooperative Patent Classification (CPC): (Cont.)
**C25B 1/50; C25B 3/07; C25B 3/26; C25B 11/091**

## Description

### Technical Field

The present application belongs to the new energy technology area, and relates to a carbon-based material and preparation thereof, particularly relates to a copper-carbon composite material, its preparation and application thereof.

### Background Art

In recent years, with the rapid development of industry and human activities, the emission amount of carbon dioxide ($CO_2$) is increased year by year, and the excessive emission of $CO_2$ is an important reason for global warming. According to statistics, the average temperature of the world in 2016-2018 is 0.9-1.1 °C higher than that in the industrialization period. The temperature rise may cause sea level rise, hazards of high tide to coastal regions, and urban flood disasters, and extreme weather may cause diseases, death, insufficient grains and the like. Therefore, $CO_2$ emission reduction situation is severe, and carbon peaking and carbon neutrality goals are put forward in China, namely arriving at a peak value of $CO_2$ emission in 2030, and achieving a carbon neutralization in 2060. To achieve the above goals, it is necessary to convert the economy relying on high consumption and high emission mainly into intensive economy on the one hand, and to develop $CO_2$ trapping and recycling technology on the other hand. One of the ways of $CO_2$ conversion and utilization is the hydro-conversion of $CO_2$ into high energy density organic fuels, such as carbon monoxide, methane, formic acid, formaldehyde, methanol, and other low carbon energy. Among them, methanol is an important chemical raw material, and can be used for synthesizing various chemical products, and can also be used as a clean substitute of fossil fuels, such as methanol gasoline, methanol fuel cells and the like. Therefore, $CO_2$ hydrogenation to produce methanol has become a focus of research interest in recent years. Currently used catalysts for producing methanol by hydrogenation of $CO_2$ are mainly copper-based catalysts. Copper species are considered to be capable of catalyzing the selective hydrogenation of carbon-oxygen bond well, and thus are widely applied to the reaction of synthesizing methanol by hydrogenation. Existing commercial catalysts include Cu/ZnO-Al$_2$CO$_3$, but said catalyst has poor catalytic activity and stability.

CN Patent No. CN112121805A discloses a catalyst for synthesizing methanol by hydrogenation of carbon dioxide, and its preparation and application, wherein a certain amount of alcohol solvent is added into copper salt, zinc salt and zirconium salt, and the mixture is stirred and ultrasonically dispersed, and then transferred into a reaction kettle to perform solvothermal reaction under a sealed condition, to obtain the catalyst for producing methanol by hydrogenation of carbon dioxide. CN Patent No. CN111215084A discloses a copper-based catalyst for producing methanol by hydrogenation of carbon dioxide, and its preparation and application, wherein the catalyst comprises copper, zinc and aluminum as active components, the zinc and aluminum components are first precipitated, aged and calcined at low temperature to obtain a zinc-aluminum hydrotalcite precursor with higher stability and more beneficial to copper dispersity, then the active component copper and the precursor are subjected to deposition and precipitation reaction to finally obtain a novel copper-zinc-aluminum catalyst suitable for producing methanol by hydrogenation of carbon dioxide.

At present, copper-based catalysts prepared by existing method are usually a bulk phase catalyst, of which the copper consumption is large, the corresponding copper utilization rate is low, and the economic cost is high, so that the large-scale industrial application of such copper-based catalysts is limited. Supported catalysts are receiving more and more extensive attention from researchers due to their characteristics, such as high dispersion, low content, and high effective utilization rate of metal component, and the like, and therefore the development of a metal supported catalytic material is one of the problems urgently needed to be solved at present.

### Summary of the Invention

To overcome the defects in the prior art, an object of the present application is to provide a copper-carbon composite material, its preparation and application thereof. The inventors of the present application have found that a copper-carbon composite material having high catalytic activity and high stability can be produced by preliminarily dispersing a copper active component on a carbonaceous material carrier, then forming pore channels in situ in the carrier, and further dispersing the copper active component into the newly formed pore channels. The present application has been completed based on this finding.

In an aspect, the present application is directed to a copper-carbon composite material, comprising an active component and a carrier, wherein the active component comprises a combination of Cu and Cu$_2$O, the carrier is a porous carbonaceous material, the combination is present in an amount, calculated by mass on the basis of the Cu element, of 1wt% to 50wt%, preferably 5wt% to 35wt%, relative to the composite material as 100 wt%, and the R1 value of the composite material is 0.4-2 : 1, preferably 0.5-1.5 : 1, wherein the R1 value is the height ratio between the peaks for Cu$_2$O and Cu in an XRD pattern of the composite material.

In another aspect, the present application is directed to a method for producing a copper-carbon composite material, comprising the steps of:

(1) contacting a carbonaceous material, a copper-containing compound and an organic solvent under heat treatment conditions to obtain a pre-composite material (referred to as material A);
(2) contacting the material A with an alkali activator to carry out an activation reaction to obtain the copper-carbon composite material,

wherein the carbonaceous material is a solid carbon-based material with a carbon content of more than 80wt% and is at least one selected from the group consisting of graphite precursors and activated carbon precursors.

In still another aspect, the present application is directed to the use of the copper-carbon composite material according to the present application in the preparation of methanol by hydrogenation of carbon dioxide, the adsorption separation of CO, the preparation of light hydrocarbons by hydrogenation of carbon dioxide, the photocatalytic conversion of carbon dioxide, the electrocatalytic conversion of carbon dioxide, and the like.

**Technical Effects**

Compared with the prior art, the copper-carbon composite material and the preparation and application thereof have one of the following advantages or the combination of part or all of them:

1. According to the present application, a composite material with an active component comprising both copper element present in the valence state of $Cu$ and copper element present in the valence state of $Cu_2O$ can be generated in situ, and the composite material has the advantages of good dispersion of active metal, desirable specific surface area, concentrated pore size distribution, high reaction activity and the like, and simplicity in preparation.

2. According to the present application, a copper-carbon composite material with a controllable and stable $Cu/Cu_2O$ ratio can be obtained, which shows a high selectivity for producing methanol by hydrogenation of $CO_2$.

3. According to the present application, the precursor such as petroleum coke and the like and the copper-containing compound are first pretreated in the presence of an organic solvent, so that the contact between the copper-containing compound and the precursor can be effectively enhanced through the infiltration swelling effect of the polar organic solvent, uniform dispersion of the copper-containing compound on the precursor is promoted, subsequent activation reaction of the precursor is facilitated, and the pretreatment using the organic solvent can be more beneficial to the simultaneous generation of $Cu$ and $Cu_2O$.

4. According to the present application, the active component copper is introduced in situ during the activation process of the precursor, wherein diffusion paths into graphite crystallite lamellas and amorphous defects of the precursor are generated through pore forming by an activator, and the copper active component enters porous carbon pore channels of the precursor along with the molten activator to form a high-dispersion structure. In addition, the use of a lower activation temperature in the present application leads to desirable specific surface area and pore distribution, and is beneficial to the simultaneously generation of active components $Cu$ and $Cu_2O$.

5. According to the present application, when the copper-carbon composite material is used in catalysts for producing methanol by hydrogenation of carbon dioxide, the porous carbon-based material used as a carrier provides abundant specific surface and pore channels, and is a structure promoter for loading active components in a high-dispersion manner; meanwhile, the graphite microcrystallite layers of the porous carbon-based material also provides a channel for electron transfer of the active center, and is a good conductive agent, so that the catalytic action of the $Cu$ and $Cu_2O$ active centers can be better exerted, and the reaction effect of producing methanol by hydrogenation of carbon dioxide can be improved.

**Brief Description of the Drawings**

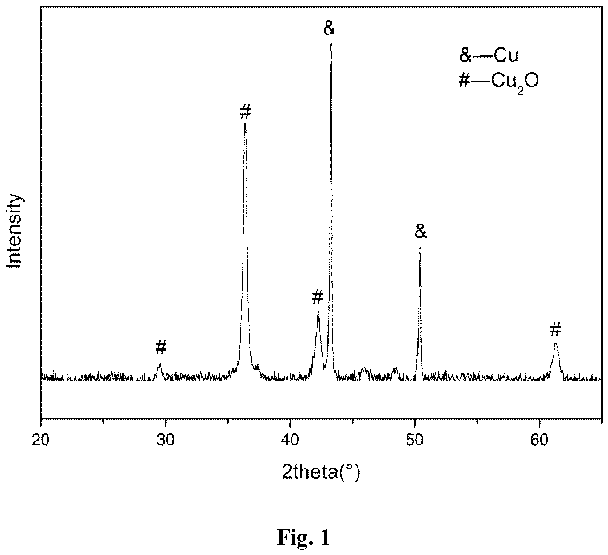

Fig. 1 is an XRD pattern of the copper-carbon composite material obtained in Example 1 of the present application.

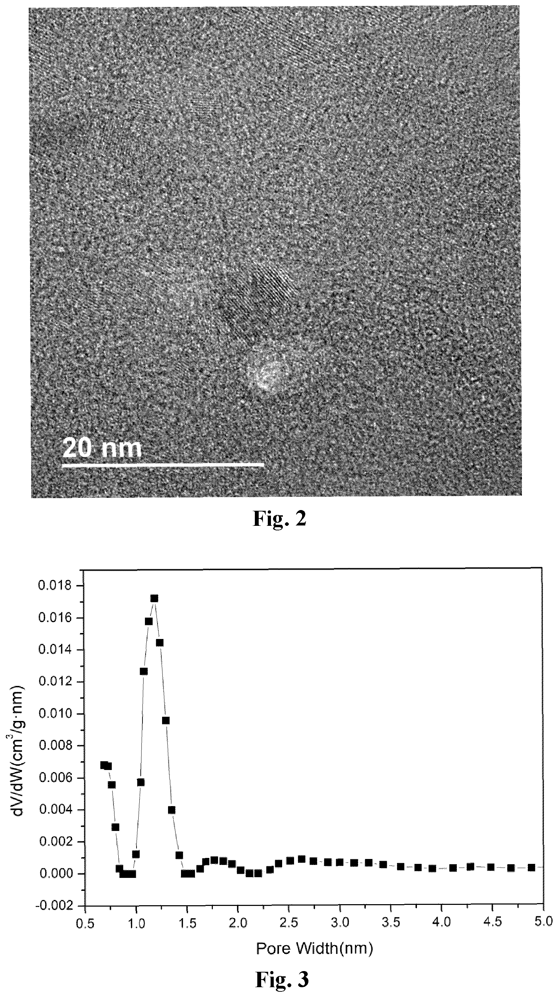

Fig. 2 is a TEM image of the copper-carbon composite material obtained in Example 1 of the present application.
Fig. 3 is a pore distribution diagram of the copper-carbon composite material obtained in Example 1 of the present application.

**Detailed Description of the Invention**

The present application will be illustrated in detail hereinbelow with reference to embodiments thereof, but it should be noted that the scope of the present application is not limited by those embodiments, but is defined by the appended claims.

All publications, patent applications, patents, and other references cited herein are incorporated by reference in their entirety. Unless defined otherwise, all technical and scientific terms used herein have the same meaning as commonly understood by those skilled in the art. In case of conflict, the contents described herein, including definitions, should prevail.

Where a material, substance, method, step, device, component, or the like is described herein as "commonly known to those skilled in the art", "prior art" or the like, it is to be understood that said material, substance, method, step, device and component cover not only those conventionally used in the art at the time of filing the present application, but also those not commonly used at present but will become commonly known in the art to be suitable for a similar purpose.

Throughout the context of the specification and claims, unless explicitly stated otherwise, the term "comprise" or its variations such as "comprises" or "comprising", etc., shall be understood as including the element or component explicitly mentioned, without excluding any other element or component.

In the context of the present application, all numerical values of a parameter (e.g., quantity or condition) shall be understood as being modified in all instances by the term "about", no matter whether or not the term "about" is actually present before the numerical value.

In the context of the present application, petroleum coke refers to solid coke produced by coking of heavy oil in a coker.

In the context of the present application, a graphite precursor refers to any carbonaceous material capable of being processed to produce graphite or to obtain a lamellar structure similar to graphite.

In the context of the present application, activated carbon precursor refers to any carbonaceous material capable of being processed to produce activated carbon or to obtain a pore structure similar to activated carbon.

In the context of the present application, the specific surface area is measured according to the nitrogen physisorption method, using an ASAP 2460 Physisorption Apparatus from Micromeritics Instrument Corporation, under test conditions including: vacuum treating the samples at 200 °C for 5 h and performing the test at liquid nitrogen temperature (-196 °C). The adsorption-desorption isotherm is obtained by static measurement, the specific surface area of the catalyst is calculated according to the BET (Brunauer-Emmett-Teller) equation, and the pore size distribution is calculated according to the NLDFT method.

In the context of the present application, the copper content in the composite material is obtained by determining the metal content using a Thermal Scientific-IRIS Intrepid IIXSP Inductively Coupled Plasma Atomic Emission Spectrometer and calculating according to the equation: copper content (%) = mass of copper/mass of the composite material * 100%.

In the context of the present application, the grain size of the active component in the composite material is calculated from the XRD results using the Scherrer formula.

In the context of the present application, the mass ratio of $Cu_2O/Cu$ in the composite material is obtained by determining using a combination of X-ray photoelectron spectroscopy (XPS) and Auger electron spectroscopy (XAES) and calculating, wherein the instrument used is a Multilab 2000 X-ray photoelectron spectrometer from Thermo Fisher Scientific, USA, the electron binding energy (B.E.) value is calibrated for the charging effect of the sample with C 1s = 284.6 eV as an internal standard, the surface atomic concentration ratio is calculated based on the peak area, and calibrated with the atomic sensitivity factor.

In the context of the present application, the occurrence state of the active component is characterized by transmission electron microscopy, TEM, under test conditions including: (i) producing a sample: grinding the composite material into powder, taking a small amount of sample and adding thereinto absolute ethyl alcohol, dispersing the sample by ultrasonic waves, finally dropping the dispersed liquid onto a copper mesh, and allowing the ethyl alcohol to volatilize till dry for later use; and (ii) observing the sample under a transmission electron microscope.

In the context of the present application, the copper dispersity is measured by CO chemisorption under test conditions including: measuring using an Autochem 2910 Chemisorption Apparatus from Micromeritics Instrument Corporation, and detecting using a thermal conductivity cell detector (TCD). The detailed procedure includes: (i) pre-reduction of catalyst: charging about 0.1 g of the composite material into a U-shaped quartz reactor, first reducing with $H_2$ for 1 h at 350 °C, then purging with He gas at 360 °C for 1 h, and then cooling to 50 °C; (ii) CO pulse adsorption: performing a CO pulse adsorption experiment after the baseline becoming stable, using 5% CO/He as an adsorbate until adsorption saturation; and (iii) CO desorption: performing a temperature programmed desorption experiment under He atmosphere at a heating rate of 10 °C/min to 900 °C. Then, the adsorption amount is calculated based on the peak area. The copper dispersity = CO adsorption amount (g)/copper content in the composite material (g) * 100%.

In the context of the present application, the carbon content is measured according to the elemental analysis method under test conditions including: conducting an experiment on a Vario MICRO Element Analyzer by subjecting a sample to high-temperature combustion oxidation in the analyzer, and separating the resulted gas to measure the carbon content.

In the context of the present application, unless specifically stated otherwise, all percentages, parts, ratios, etc. are expressed by weight and all pressures given are gauge pressures.

In the context of the present application, any two or more embodiments of the present application may be arbitrarily combined, and the resulting technical solution forms a part of the initial disclosure of the present application and falls within the scope of the present application.

According to an embodiment of the present application, a copper-carbon composite is provided. According to the present application, the copper-carbon composite material is particularly suitable for use as a catalyst, and thus it is sometimes simply referred to as a catalyst in the present specification.

According to an embodiment of the present application, the copper-carbon composite material comprises an active component and a carrier.

According to an embodiment of the present application, the active component comprises a combination of Cu and $Cu_2O$. The active component may also comprise further components in order to adjust the catalyst properties or to meet the requirements of the application.

According to an embodiment of the present application, the carrier is a porous carbonaceous material. Preferably, the porous carbonaceous material is a solid carbon-based material having a carbon content of more than 80 wt%, preferably derived from at least one selected from petroleum coke, needle coke, pitch, biomass char and coal, more preferably derived from petroleum coke.

According to an embodiment of the present application, the content of said combination, calculated based on the mass of the Cu element, is between 1% and 50% by weight, preferably between 5% and 35% by weight, relative to 100% by weight of said composite material.

According to an embodiment of the present application, the R1 value of the composite material is 0.4-2 : 1, preferably 0.5-1.5 : 1, wherein the R1 value is the height ratio between the peaks for $Cu_2O$ and Cu in the XRD pattern of the composite material. According to the present application, the composite material has a higher $Cu_2O$ percentage, as compared to existing catalysts. If the R1 value in the copper-carbon composite material is lower or higher than the above value, the effect of catalyzing hydrogenation of $CO_2$ for producing methanol is poor.

According to a preferred embodiment of the present application, the copper-carbon composite has an R2 value of 0.05-0.4 : 1, preferably 0.05-0.3 : 1, wherein the R2 value is the area ratio between the peaks for $Cu^+$ and $Cu^0$ obtained by peak differentiating and imitating based on the XPS spectrum and auger electron spectroscopy (XAES) of the composite material. According to the preferred embodiment, if the R2 value of the copper-carbon composite material is higher than the above value, the catalyst has poor storage stability and is easy to be further oxidized to copper oxide; while if the R2 value is lower than the above value, and the R1 value is also low, the effect of the composite material in catalyzing hydrogenation of $CO_2$ for producing methanol is poor.

According to an embodiment of the present application, the copper-carbon composite material shows a substantially unchanged R1 value of 0.4-2 : 1, preferably 0.5-1.5 : 1, and a substantially unchanged R2 value of 0.05-0.4 : 1, preferably 0.05-0.3 : 1, when measured after being kept at 25 °C for 48 hours under an air atmosphere. This measurement result indicates that the copper-carbon composite material of the present application has very strong storage stability and the active component can be maintained substantially unchanged for a long period of time.

According to an embodiment of the present application, at least a part (preferably substantially all) of the active component is embedded into the graphite microcrystallite layers and amorphous defects of the porous carbonaceous material.

According to an embodiment of the present application, the copper dispersity is between 5% and 20%, preferably between 10% and 20%.

According to an embodiment of the present application, the grain size of the active component is between 3 nm and 20 nm, preferably between 5 nm and 15 nm.

According to an embodiment of the present application, the pore volume of pores having a pore diameter of 0.8 nm to 2 nm in the copper-carbon composite material is from 30% to 50%, preferably from 30% to 45%, of the total pore volume.

According to an embodiment of the present application, the specific surface area of the copper-carbon composite material is 100-600 $m^2/g$, and preferably 150-500 $m^2/g$.

According to an embodiment of the present application, a method for producing the copper-carbon composite material is also provided.

According to an embodiment of the present application, the method comprises the steps of:

contacting a carbonaceous material, a copper-containing compound and an organic solvent under heat treatment conditions to obtain a pre-composite material (referred to as material A);
contacting the material A with an alkali activator for activation reaction to obtain the copper-carbon composite material.

According to an embodiment of the present application, the carbonaceous material is a solid carbon-based material having a carbon content of more than 80wt%, and is at least one selected from the group consisting of graphite precursors and activated carbon precursors. Preferably, the carbonaceous material is at least one selected from the group consisting of petroleum coke, needle coke, pitch, biomass char, and coal, more preferably petroleum coke.

According to an embodiment of the present application, the method further comprises washing and drying the resultant after the activation reaction to obtain the copper-carbon composite material.

According to an embodiment of the present application, the washing is normally water washing, wherein water washing means washing with water and filtering until the filtrate has a neutral pH.

According to an embodiment of the present application, the drying may be atmospheric drying or vacuum drying, and preferably vacuum drying.

According to an embodiment of the present application, the drying temperature is 50 °C to 250 °C, preferably 60 °C to 150 °C. The drying time is 2-24 h, preferably 5-16 h.

According to an embodiment of the present application, the copper-containing compound is one or more selected from the group consisting of inorganic copper salt, organic copper salt, copper-containing oxide, and copper-containing hydroxide, specifically one or more selected from the group consisting of copper nitrate, basic copper carbonate, copper sulfate, copper chloride, copper formate, copper acetate, copper hydroxide, and copper oxide, and preferably one or more selected from the group consisting of copper chloride, copper nitrate, basic copper carbonate, copper sulfate, copper formate, and copper acetate.

According to an embodiment of the present application, the organic solvent is one or more selected from the group consisting of pyrrolidone derivatives, N,N-dimethylformamide, methanol, ethanol, tetrahydrofuran, and carbon disulfide, preferably pyrrolidone derivatives, more preferably at least one selected from N-methyl pyrrolidone and N-ethyl pyrrolidone, and particularly preferably N-methyl pyrrolidone.

According to an embodiment of the present application, in the step (1), the carbonaceous material, the copper-containing compound, and the organic solvent are uniformly mixed and subjected to the heat treatment, and then dried to obtain the material A.

According to an embodiment of the present application, the drying conditions includes: a drying temperature of 60-350 °C, preferably 80-300 °C; a drying time of 0.5-24 h, preferably 1-18 h.

According to an embodiment of the present application, the drying may be performed in any one of the existing drying manners, specifically may be performed by one or more of oven drying, vacuum drying, and rotary evaporation drying, and preferably by vacuum drying or rotary evaporation drying.

According to an embodiment of the present application, the operating conditions of the heat treatment comprise: a treatment temperature of 60 °C to 400 °C, preferably 80 °C to 350 °C, a treatment time of 0.1 to 24 hours, preferably 1 to 16 hours, and a treatment pressure (gauge pressure) of 0 MPa to 15 MPa, preferably 1 MPa to 10 MPa, and more preferably 2 MPa to 8 MPa.

According to an embodiment of the present application, the heat treatment is carried out without the introduction or addition of liquid or vaporous water, which means that no liquid or vaporous water is intentionally introduced or added during the heat treatment, apart from moisture that may be contained in the respective raw materials (i.e. the carbonaceous material, the copper-containing compound and the organic solvent), per se.

According to an embodiment of the present application, the mass ratio of the carbonaceous material, the copper-containing compound, and the organic solvent is 1 : 0.01-1.2 : 1-100, preferably 1 : 0.1-0.8 : 10-50, wherein the copper-containing compound is calculated based on the mass of the Cu element.

According to an embodiment of the present application, the alkali activator is one or more selected from the group consisting of potassium hydroxide, potassium carbonate, potassium bicarbonate, sodium hydroxide and calcium hydroxide, and preferably potassium hydroxide.

According to an embodiment of the present application, the mass ratio of the material A to the alkali activator is 1 : 0.2-12, preferably 1 : 0.5-6.

According to an embodiment of the present application, the operating conditions of the activation reaction include: an atmosphere being one or more of nitrogen and/or inert atmosphere, preferably nitrogen, an activation temperature of 300-600 °C, preferably 400-550 °C, and an activation time of 0.1-10 h, preferably 0.5-8 h. The alkali activator is typically present in a molten state during the activation reaction. Herein, the molten state means that the alkali activator is present as a liquid, but its temperature is not necessarily the melting temperature of the alkali activator.

According to an embodiment of the present application, the inert gas is one or more selected from the group of helium, argon and neon.

According to an embodiment of the present application, the activation reaction is carried out without the introduction or addition of liquid or vaporous water, which means that no liquid or vaporous water is intentionally introduced or added during the activation reaction, apart from the moisture that may be contained in the respective starting materials (i.e. material A and alkaline activator), per se.

According to an embodiment of the present application, there is also provided use of the copper-carbon composite material according to the present application or the copper-carbon composite material obtained according to the method of the present application in the production of methanol by hydrogenation of carbon dioxide.

According to an embodiment of the present application, there is also provided use of the copper-carbon composite material according to the present application or the copper-carbon composite material obtained according to the method of the present application in the adsorptive separation of CO.

According to an embodiment of the present application, there is also provided use of the copper-carbon composite material according to the present application or the copper-carbon composite material obtained by the method according to the present application in the production of lower hydrocarbons by hydrogenation of carbon dioxide.

According to an embodiment of the present application, there is also provided use of the copper-carbon composite material according to the present application or the copper-carbon composite material obtained by the method according to the present application in the photocatalytic conversion of carbon dioxide.

According to an embodiment of the present application, there is also provided use of the copper-carbon composite material according to the present application or the copper-carbon composite material obtained by the method according to the present application in the electrocatalytic conversion of carbon dioxide.

According to the present application, as the operating conditions of these applications, those known in the art can be directly used and there is no particular limitation.

## Examples

The present application will be described in further detail with reference to examples, but the present application is not limited to these examples.

The evaluation of the activity of the composite material in the reaction for producing methanol by hydrogenation of carbon dioxide was carried out on a fixed bed reactor, under the following conditions: a certain amount of catalyst was charged into a reaction tube, and heated under a nitrogen atmosphere to the reaction temperature, the atmosphere was switched to the reaction gas (the volume ratio of carbon dioxide to hydrogen was 1 : 3, and a certain amount of nitrogen was mixed as an internal standard), the gas space velocity was 3000 mL/(g•h), the reaction temperature was 250 °C, and the reaction pressure was 4 MPa. After the reaction became stable, on-line analysis was performed by gas chromatography using Agilent 8860 Gas Chromatograph, equipped with a TCD detector.

$$\text{Carbon dioxide conversion } \ C_{CO_2}\% = \frac{CO2(in) - CO2(out) \times \frac{N2(in)}{N2(out)}}{CO2(in)} \times 100\%;$$

$$\text{Methanol selectivity } \ S_{CH3OH}\% = \frac{CH3OH(out)}{CO2(in) \times \frac{N2(out)}{N2(in)} - CO2(out)} \times 100\%;$$

wherein x(in) represents the concentration of x in the feed gas, x(out) represents the concentration of x in the product gas, and x is $CO_2$, $CH_3OH$, $N_2$.

Example 1

Petroleum coke ground to a particle size of 20-300 meshes, basic copper carbonate and N-methyl pyrrolidone were uniformly mixed at a mass ratio of 1 : 0.26 : 50, treated for 8 hours under the conditions of 5 MPa and 100 °C, and cooled to room temperature. A sample was taken and placed in a vacuum drying oven for drying at a temperature of 150 °C for 10 hours. The resulted product A1 and potassium hydroxide were uniformly mixed at a mass ratio of 1 : 1.5, and activated for 3 hours at a temperature of 500 °C under a nitrogen atmosphere. After being cooled to room temperature, the activated product was filtered and washed with water until the pH value of the filtrate was neutral, the solid product was dried for 5 hours in a vacuum drying oven at 100 °C to obtain a composite material C-1. The properties of the sample are shown in Table 1, and the evaluation results are shown in Table 2.

By observing the TEM image of the composite material C-1, it can be seen that the active component is embedded into the graphite miacrocrystallite layers and the amorphous defects of the carrier material.

The composite material C-1 showed substantially no change in the R1 value and the R2 value when measured after being kept at 25 °C for 48 hours under an air atmosphere.

Example 2

Petroleum coke ground to a particle size of 20-300 meshes, copper chloride and N-ethyl pyrrolidone were uniformly mixed at a mass ratio of 1 : 0.28 : 20, treated for 3h under the conditions of 2 MPa and 100 °C, and cooled to room temperature. A sample was taken and placed in a vacuum drying oven for drying at 180 °C for 6h. The resulted product A2 and potassium hydroxide were uniformly mixed at a mass ratio of 1 : 1, and activated for 4 h under a nitrogen atmosphere at 500 °C. After being cooled to room temperature, the activated product was filtered and washed with water until the pH value of the filtrate was neutral, the solid product was dried in a vacuum drying oven at 120 °C for 4h to obtain a composite material C-2. The properties of the sample are shown in Table 1, and the evaluation results are shown in Table 2.

Example 3

Petroleum coke ground to a particle size of 20-300 meshes, basic copper carbonate and N-methyl pyrrolidone were uniformly mixed at a mass ratio of 1 : 0.42 : 40, treated for 3h under the conditions of 4 MPa and 200 °C, and cooled to room temperature. A sample was taken and placed in a vacuum drying oven for drying at a temperature of 150 °C for 8h. The resulted product A3 and potassium hydroxide were uniformly mixed at a mass ratio of 1 : 1.5, and activated for 4h under a nitrogen atmosphere at a temperature of 490 °C. After being cooled to room temperature, the activated product was filtered and washed with water until the pH value of the filtrate was neutral, the solid product was dried for 6 hours in a vacuum drying oven at 120 °C to obtain a composite material C-3. The properties of the sample are shown in Table 1, and the evaluation results are shown in Table 2.

Example 4

Petroleum coke ground to a particle size of 20-300 meshes, basic copper carbonate and N-methyl pyrrolidone were uniformly mixed at a mass ratio of 1 : 0.13 : 30, treated for 10 hours under the conditions of 5 MPa and 150 °C, and cooled to room temperature. A sample was taken and placed in a vacuum drying oven for drying at a temperature of 200 °C for 6 hours. The resulted product A4 and potassium hydroxide were uniformly mixed at a mass ratio of 1 : 2, and activated for 4 hours at a temperature of 450 °C under a nitrogen atmosphere. After being cooled to room temperature, the activated product was filtered and washed with water until the pH value of the filtrate was neutral, the solid product was dried in a vacuum drying oven at 100 °C for 8 hours to obtain a composite material C-4. The properties of the sample are shown in Table 1, and the evaluation results are shown in Table 2.

Example 5

Petroleum coke ground to a particle size of 20-300 meshes, copper nitrate and N-methyl pyrrolidone were uniformly mixed at a mass ratio of 1 : 0.68 : 30, treated for 6h under the conditions of 7 MPa and 110 °C, and cooled to room temperature. A sample was taken and placed in a vacuum drying oven for drying at a temperature of 150 °C for 6h. The resulted product A5 and potassium hydroxide were uniformly mixed at a mass ratio of 1 : 0.8, and activated for 2h under a nitrogen atmosphere at a temperature of 500 °C. After being cooled to room temperature, the activated product was filtered and washed with water until the pH value of the filtrate was neutral, the solid product was dried for 7 hours at 120 °C in a vacuum drying oven to obtain a composite material C-5. The properties of the sample are shown in Table 1, and the evaluation results are shown in Table 2.

Example 6

Petroleum coke ground to a particle size of 20-300 meshes, copper chloride and N-methyl pyrrolidone were uniformly mixed at a mass ratio of 1 : 0.56 : 15, treated for 10 hours under the conditions of 2 MPa and 100 °C, and cooled to room temperature. A sample was taken and placed in a vacuum drying oven for drying at a temperature of 200 °C for 5 hours. The resulted product A6 and potassium hydroxide were uniformly mixed at a mass ratio of 1 : 1, and activated for 6 hours at a temperature of 450 °C under a nitrogen atmosphere. After being cooled to room temperature, the activated product was filtered and washed with water until the pH value of the filtrate was neutral, the solid product was dried for 5 hours in a vacuum drying oven at 130 °C to obtain a composite material C-6. The properties of the sample are shown in Table 1, and the evaluation results are shown in Table 2.

Example 7

Petroleum coke ground to a particle size of 20-300 meshes, basic copper carbonate and N-methyl pyrrolidone were uniformly mixed at a mass ratio of 1 : 0.49 : 40, treated for 2 hours under the conditions of 10 MPa and 100 °C, and cooled to room temperature. A sample was taken and placed in a vacuum drying oven for drying at a temperature of 150 °C for 8 hours. The resulted product A7 and potassium hydroxide were uniformly mixed at a mass ratio of 1 : 1.5, and activated for 3 hours at a temperature of 500 °C under a nitrogen atmosphere. After being cooled to room temperature, the activated product was filtered and washed with water until the pH value of the filtrate was neutral, the solid product was dried for 6 hours in a vacuum drying oven at 120 °C to obtain a composite material C-7. The properties of the sample are shown in Table 1, and the evaluation results are shown in Table 2.

Example 8

Petroleum coke ground to a particle size of 20-300 meshes, copper chloride and N-methyl pyrrolidone were uniformly mixed at a mass ratio of 1 : 0.15 : 20, treated for 2 hours under the conditions of 3 MPa and 100 °C, and cooled to room temperature. A sample was taken and placed in a vacuum drying oven for drying at 180 °C for 4 hours. The resulted product A8 and potassium hydroxide were uniformly mixed at a mass ratio of 1 : 2, and activated for 4 hours at 525 °C under a nitrogen atmosphere. After being cooled to room temperature, the activated product was filtered and washed with water until the pH value of the filtrate was neutral, the solid product was dried for 6 hours in a vacuum drying oven at 110 °C to obtain a composite material C-8. The properties of the sample are shown in Table 1, and the evaluation results are shown in Table 2.

Example 9

Petroleum coke ground to a particle size of 20-300 meshes, basic copper carbonate and N-methyl pyrrolidone were uniformly mixed at a mass ratio of 1 : 0.19 : 45, treated for 4 hours under the conditions of 6 MPa and 250 °C, and cooled to room temperature. A sample was taken and placed in a vacuum drying oven for drying at a temperature of 150 °C for 4 hours. The resulted product A9 and potassium hydroxide were uniformly mixed at a mass ratio of 1 : 2, and activated for 2 hours at a temperature of 500 °C under a nitrogen atmosphere. After being cooled to room temperature, the activated product was filtered and washed with water until the pH value of the filtrate was neutral, the solid product was dried in a vacuum drying oven at 100 °C for 8 hours to obtain a composite material C-9. The properties of the sample are shown in Table 1, and the evaluation results are shown in Table 2.

Example 10

Petroleum coke ground to a particle size of 20-300 meshes, copper nitrate and N-methyl pyrrolidone were uniformly mixed at a mass ratio of 1 : 0.42 : 40, treated for 2 hours under the conditions of 5 MPa and 350 °C, and cooled to room temperature. A sample was taken and placed in a vacuum drying oven for drying at 180 °C for 4 hours. The resulted product A10 and potassium hydroxide were uniformly mixed at a mass ratio of 1 : 3, and activated for 4 hours at 450 °C under a nitrogen atmosphere. After being cooled to room temperature, the activated product was filtered and washed with water until the pH value of the filtrate was neutral, the solid product was dried in a vacuum drying oven at 120 °C for 6 hours to obtain a composite material C-10. The properties of the sample are shown in Table 1, and the evaluation results are shown in Table 2.

Example 11

Petroleum coke ground to a particle size of 20-300 meshes, copper chloride and N,N-dimethylformamide were uniformly mixed at a mass ratio of 1 : 0.15 : 18, treated for 3 hours under the conditions of 2 MPa and 90 °C, and cooled to room temperature. A sample was taken and placed in a vacuum drying oven for drying at a temperature of 140 °C for 8 hours. The resulted product A11 and potassium hydroxide were uniformly mixed at a mass ratio of 1 : 2, and activated for 3 hours under a nitrogen atmosphere at a temperature of 500 °C. After being cooled to room temperature, the activated product was filtered and washed with water until the pH value of the filtrate was neutral, the solid product was dried in a vacuum drying oven for 6 hours at 110 °C to obtain a composite material C-11. The properties of the sample are shown in Table 1, and the evaluation results are shown in Table 2.

Example 12

Coal ground to a particle size of 20-300 meshes, copper nitrate and N-ethyl pyrrolidone were uniformly mixed at a mass ratio of 1 : 0.33 : 40, treated for 4 hours under the conditions of 3 MPa and 120 °C, and cooled to room temperature. A sample was taken and placed into a vacuum drying oven for drying at a temperature of 150 °C for 6 hours. The resulted product A12 and potassium hydroxide were uniformly mixed at a mass ratio of 1 : 1.5, and activated for 5 hours at a temperature of 450 °C under a nitrogen atmosphere. After being cooled to room temperature, the activated product was filtered and washed with water until the pH value of the filtrate was neutral, the solid product was dried in a vacuum drying oven at 120 °C for 12 hours to obtain a composite material C-12. The properties of the sample are shown in Table 1, and the evaluation results are shown in Table 2.

Example 13

Petroleum coke ground to a particle size of 20-300 meshes, copper nitrate and N-methyl pyrrolidone were uniformly mixed at a mass ratio of 1 : 0.36 : 40, treated for 8 hours at a temperature of 130 °C under normal pressure, and cooled to room temperature. A sample was taken and placed in a vacuum drying oven for drying at a temperature of 180 °C for 5 hours. The resulted product A13 and potassium hydroxide were uniformly mixed at a mass ratio of 1 : 3, and activated for 4 hours at a temperature of 475 °C under a nitrogen atmosphere. After being cooled to room temperature, the activated product was filtered and washed with water until the pH value of the filtrate was neutral, the solid product was dried in a vacuum drying oven for 18 hours at 110 °C to obtain a composite material C-13. The properties of the sample are shown in Table 1, and the evaluation results are shown in Table 2.

Example 14

Petroleum coke ground to a particle size of 20-300 meshes, basic copper carbonate and N-methyl pyrrolidone were uniformly mixed at a mass ratio of 1 : 0.09 : 9, treated for 5 hours under the conditions of 1 MPa and 100 °C, and cooled to room temperature. A sample was taken and placed in a vacuum drying oven for drying at a temperature of 120 °C for 10 hours. The resulted product A14 and potassium hydroxide were uniformly mixed at a mass ratio of 1 : 1.5, and activated for 3 hours at a temperature of 500 °C under a nitrogen atmosphere. After being cooled to room temperature, the activated product was filtered and washed with water until the pH value of the filtrate was neutral, the solid product was dried in a vacuum drying oven at 100 °C for 18 hours to obtain a composite material C-14. The properties of the sample are shown in Table 1, and the evaluation results are shown in Table 2.

Example 15

Petroleum coke ground to a particle size of 20-300 meshes, basic copper carbonate and N-methyl pyrrolidone were uniformly mixed at a mass ratio of 1 : 0.13 : 15, treated for 3h under the conditions of 2 MPa and 100 °C, and cooled to room temperature. A sample was taken and placed in a vacuum drying oven for drying at a temperature of 130 °C for 8h. The resulted product A15 and potassium hydroxide were uniformly mixed at a mass ratio of 1 : 7, and activated for 6h under a nitrogen atmosphere at a temperature of 450 °C. After being cooled to room temperature, the activated product was filtered and washed with water until the pH value of the filtrate was neutral, the solid product was dried in a vacuum drying oven at 130 °C for 10 hours to obtain a composite material C-15. The properties of the sample are shown in Table 1, and the evaluation results are shown in Table 2.

Comparative Example 1

Activated carbon with a specific surface area of 421 $m^2/g$ and copper chloride were uniformly mixed at a mass ratio of 1 : 0.5, calcined for 6 hours at 550 °C under a nitrogen atmosphere, and cooled to room temperature. The resulted product was filtered and washed with water until the pH value of the filtrate was neutral, the solid product was dried in a vacuum drying oven for 6 hours at 100 °C to obtain a composite material D-1. The properties of the sample are shown in Table 1, and the evaluation results are shown in Table 2.

Comparative Example 2

Petroleum coke ground to a particle size of 20-300 meshes, copper nitrate and N-methyl pyrrolidone were uniformly mixed at a mass ratio of 1 : 0.42 : 40, treated for 2 hours at 350 °C under normal pressure, and cooled to room temperature. A sample was taken and placed in a vacuum drying oven for drying at 180 °C for 4 hours. The resulted product B2 and potassium hydroxide were uniformly mixed at a mass ratio of 1 : 3, and activated for 4 hours at 450 °C under a nitrogen atmosphere. After being cooled to room temperature, the activated product was filtered and washed with water until the pH value of the filtrate was neutral, the solid product was dried in a vacuum drying oven at 120 °C for 6 hours to obtain a composite material D-2. The properties of the sample are shown in Table 1, and the evaluation results are shown in Table 2.

Comparative Example 3

Petroleum coke ground to a particle size of 20-300 meshes, basic copper carbonate and potassium hydroxide were uniformly mixed at a mass ratio of 1 : 0.33 : 1, and activated for 3 hours at 500 °C under a nitrogen atmosphere. After being cooled to room temperature, the activated product was filtered and washed with water until the pH value of the filtrate was neutral, the solid product was dried for 5 hours in a vacuum drying oven at 100 °C to obtain a composite material D-3.

The properties of the sample are shown in Table 1, and the evaluation results are shown in Table 2.

Comparative Example 4

Petroleum coke ground to a particle size of 20-300 meshes and copper nitrate were uniformly mixed at a mass ratio of 1 : 0.42 : 40, treated for 2 hours under the conditions of 5 MPa and 350 °C, and cooled to room temperature. A sample was taken and placed in a vacuum drying oven for drying at 180 °C for 4 hours. The resulted product B4 and potassium hydroxide were uniformly mixed at a mass ratio of 1 : 3, and activated for 4 hours at 450 °C under a nitrogen atmosphere. After being cooled to room temperature, the activated product was filtered and washed with water until the pH value of the filtrate was neutral, the solid product was dried in a vacuum drying oven at 120 °C for 6h to obtain a composite material D-4. The properties of the sample are shown in Table 1, and the evaluation results are shown in Table 2.

Comparative Example 5

Petroleum coke ground to a particle size of 20-300 meshes and copper nitrate were uniformly mixed at a mass ratio of 1 : 0.42, treated for 2 hours at 350 °C under normal pressure, and cooled to room temperature. A sample was taken and placed in a vacuum drying oven for drying at 180 °C for 4 hours. The resulted product B5 and potassium hydroxide were uniformly mixed at a mass ratio of 1 : 3, and activated for 4 hours at 450 °C under a nitrogen atmosphere. After being cooled to room temperature, the activated product was filtered and washed with water until the pH value of the filtrate was neutral, the solid product was dried in a vacuum drying oven at 120 °C for 6 hours to obtain a composite material D-5. The properties of the sample are shown in Table 1, and the evaluation results are shown in Table 2.

Comparative Example 6

1.32 g of polyethyleneimine was weighed, 100mL of ultrapure water was added, and the mixture was stirred until completely dissolved. Then, 1.97 g of copper nitrate was added thereto, followed by stirring till uniform. Then, 2g of petroleum coke activated carbon was weighed, uniformly stirred and then charged into an autoclave, the autoclave was sealed, and a hydrothermal reaction was carried out for 3 hours at a temperature of 220 °C. After the autoclave being naturally cooled to room temperature, the solid product was washed with deionized water several times, and dried in vacuum at a temperature of 60 °C to obtain a composite material D-6.

Comparative Example 7

100g of petroleum coke ground to a particle size of 20-300 meshes was weighed, 100g of petroleum coke was ground to powder, and then uniformly mixed with 46.83 g of sodium tetrachlorocuprate and 300g of potassium bicarbonate, the mixture was placed in a microwave heating furnace operating with a microwave frequency of 2450 MHz, and heated to 900 °C under a nitrogen atmosphere with a microwave power of 0.3 kw for activation for 20 min. After activation, the temperature was reduced to 300 °C under a nitrogen atmosphere, and 5% (volume fraction) $O_2$/Ar mixed gas was introduced for a treatment of 30 min. An appropriate amount of an aqueous glucose solution having a concentration of 20wt% was weighed, added to the sample obtained above, and stirred in a water bath at 90 °C for 20 min. The resulted sample was ground into powder, weighed, and mixed with deionized water at a mass ratio of 1 : 15, fully stirred, then subjected to solid-liquid separation until the pH value of the filtrate was neutral, the resulted solid sample was placed in a vacuum drying oven, and dried for 6 hours at 150 °C in vacuum to obtain a composite material D-7.

Comparative Example 8

Petroleum coke ground to a particle size of 20-300 meshes, copper nitrate and water were uniformly mixed at a mass ratio of 1 : 0.21 : 18, treated for 3 hours under the conditions of 2 MPa and 90 °C, and cooled to room temperature. A sample was taken and placed in a vacuum drying oven for drying at a temperature of 150 °C for 6 hours. The resulted product B8 and potassium hydroxide were uniformly mixed at a mass ratio of 1 : 2, and activated for 6 hours at a temperature of 500 °C under a nitrogen atmosphere. After being cooled to room temperature, the activated product was filtered and washed with water until the pH value of the filtrate was neutral, the solid product was dried for 15 hours in a vacuum drying oven at 110 °C to obtain a composite material D-8. The properties of the sample are shown in Table 1, and the evaluation results are shown in Table 2.

Comparative Example 9

Graphite ground to a particle size of 20-300 meshes, copper nitrate and N-methyl pyrrolidone were uniformly mixed at a mass ratio of 1 : 0.42 : 40, treated for 2h under the conditions of 5 MPa and 350 °C, and cooled to room temperature. A sample was taken and placed in a vacuum drying oven for drying at a temperature of 150 °C for 6h. The resulted product B9 and potassium hydroxide were uniformly mixed at a mass ratio of 1 : 3, and activated for 10h under a nitrogen atmosphere at a temperature of 500 °C. After being cooled to room temperature, the activated product was filtered and washed with water until the pH value of the filtrate was neutral, the solid product was dried for 15 hours in a vacuum drying oven at 120 °C to obtain a composite material D-9. The properties of the sample are shown in Table 1, and the evaluation results are shown in Table 2.

Comparative Example 10

Petroleum coke ground to a particle size of 20-300 meshes, copper chloride and N-methyl pyrrolidone were uniformly mixed at a mass ratio of 1 : 0.21 : 20, treated for 2 hours under the conditions of 3 MPa and 100 °C, and cooled to room temperature. A sample was taken and placed in a vacuum drying oven for drying at a temperature of 150 °C for 6 hours. The resulted product B10 was placed in a tubular furnace, with an introduction of steam, and activated for 4 hours at a temperature of 525 °C under a nitrogen atmosphere. After being cooled to room temperature, the activated product was filtered and washed with water until the pH value of the filtrate was neutral, the solid product was dried in a vacuum drying oven at 140 °C for 8 hours to obtain a composite material D-10. The properties of the sample are shown in Table 1, and the evaluation results are shown in Table 2.

Table 1 Properties of composite materials obtained in examples and comparative examples

| Sample | Composition of active components | Copper content, wt% | R1 value | R2 value | Copper dispersity (%) | Grain size of active phase (nm) | Specific surface area $(m^2/g)$ | Proportion of pores of 0.8-2 nm (%) |
|---|---|---|---|---|---|---|---|---|
| C-1 | $Cu_2O/Cu$ | 15.8 | 0.71 | 0.22 | 16.4 | 6.4 | 381 | 35.4 |
| C-2 | $Cu_2O/Cu$ | 13.0 | 0.53 | 0.15 | 12.8 | 7.6 | 284 | 36.5 |
| C-3 | $Cu_2O/Cu$ | 22.8 | 0.62 | 0.2 | 11.1 | 12.0 | 274 | 38.1 |
| C-4 | $Cu_2O/Cu$ | 9.0 | 1.02 | 0.25 | 13.9 | 6.2 | 263 | 35.1 |
| C-5 | $Cu_2O/Cu$ | 16.5 | 0.81 | 0.13 | 10.6 | 12.8 | 194 | 42.2 |
| C-6 | $Cu_2O/Cu$ | 22.9 | 0.51 | 0.14 | 11.0 | 11.4 | 267 | 33.6 |
| C-7 | $Cu_2O/Cu$ | 25.6 | 0.65 | 0.23 | 12.5 | 10.4 | 350 | 36.1 |
| C-8 | $Cu_2O/Cu$ | 9.1 | 0.61 | 0.21 | 19.3 | 8.6 | 451 | 35.1 |
| C-9 | $Cu_2O/Cu$ | 12.9 | 0.82 | 0.24 | 16.7 | 6.2 | 369 | 42.9 |
| C-10 | $Cu_2O/Cu$ | 17.2 | 0.78 | 0.30 | 12.1 | 9.1 | 285 | 38.2 |
| C-11 | $Cu_2O/Cu$ | 8.2 | 0.45 | 0.22 | 18.2 | 16.2 | 375 | 34.2 |
| C-12 | $Cu_2O/Cu$ | 12.2 | 0.48 | 0.18 | 11.8 | 12.9 | 212 | 33.9 |
| C-13 | $Cu_2O/Cu$ | 15.2 | 0.43 | 0.3 | 15.2 | 12.4 | 329 | 34.3 |
| C-14 | $Cu_2O/Cu$ | 6.5 | 0.4 | 0.21 | 18.7 | 14.2 | 336 | 35.3 |
| C-15 | $Cu_2O/Cu$ | 13.0 | 0.52 | 0.12 | 15.9 | 15.2 | 348 | 45.8 |
| D-1 | Cu | 17.9 | 0 | 0 | 11.2 | 10.8 | 421 | 31.2 |
| D-2 | $Cu_2O/Cu$ | 20.1 | 0.31 | 0.19 | 12.0 | 18.2 | 351 | 30.9 |
| D-3 | $Cu_2O/Cu$ | 12.7 | 0.29 | 0.15 | 15.4 | 20.6 | 343 | 30.1 |
| D-4 | $Cu_2O/Cu$ | 21.0 | 0.28 | 0.16 | 11.3 | 21.4 | 335 | 30.5 |
| D-5 | $Cu_2O/Cu$ | 21.2 | 0.25 | 0.14 | 10.6 | 21.1 | 341 | 30.1 |
| D-6 | $Cu_2O/Cu$ | 34.3 | 0.39 | 0.40 | 5.8 | 23.2 | 322 | 29.5 |
| D-7 | $Cu_2O/Cu$ | 19.6 | 0.21 | 0.37 | 40.5 | 14.6 | 2048 | 31.3 |
| D-8 | $Cu_2O/Cu$ | 8.2 | 0.2 | 0.05 | 18.1 | 16.2 | 381 | 34.2 |

(continued)

| Sample | Composition of active components | Copper content, wt% | R1 value | R2 value | Copper dispersity (%) | Grain size of active phase (nm) | Specific surface area ($m^2$/g) | Proportion of pores of 0.8-2 nm (%) |
|---|---|---|---|---|---|---|---|---|
| D-9 | Cu | 17.4 | 0 | 0 | - | 366 | 2 | 0 |
| D-10 | Cu | 9.1 | 0 | 0 | - | 292 | 5 | 0 |

Table 2 Evaluation results of composite materials obtained in examples and comparative examples

| Sample | $CO_2$ conversion (%) | $CH_3OH$ selectivity (%) |
|---|---|---|
| C-1 | 18.7 | 64.3 |
| C-2 | 18.4 | 62.7 |
| C-3 | 17.9 | 63.6 |
| C-4 | 17.7 | 62.4 |
| C-5 | 18.9 | 63 |
| C-6 | 18.1 | 62.2 |
| C-7 | 18.3 | 64.5 |
| C-8 | 17.6 | 62.1 |
| C-9 | 18.2 | 63.2 |
| C-10 | 17.9 | 63.1 |
| C-11 | 17.1 | 58.3 |
| C-12 | 17.1 | 60.1 |
| C-13 | 17.5 | 61.2 |
| C-14 | 17.0 | 59.0 |
| C-15 | 17.2 | 60.3 |
| D-1 | 16.8 | 47.2 |
| D-2 | 16.5 | 54.9 |
| D-3 | 16.2 | 52.7 |
| D-4 | 16.3 | 53.2 |
| D-5 | 16.2 | 52.9 |
| D-6 | 16.4 | 49.8 |
| D-7 | 16.9 | 50.3 |
| D-8 | 16.1 | 53.5 |
| D-9 | 0.5 | 31.0 |
| D-10 | 1.0 | 35.1 |

**Claims**

1. A copper-carbon composite material, comprising an active component and a carrier, wherein the active component comprises a combination of Cu and $Cu_2O$, and the carrier is a porous carbonaceous material, the combination is present in an amount of 1 wt% to 50 wt%, preferably 5 wt% to 35 wt%, calculated by mass on the basis of Cu element, relative to 100 wt% of the composite material, the composite material has a R1 value of 0.4-2 : 1, preferably 0.5-1.5 : 1, wherein the R1 value is the height ratio between peaks for $Cu_2O$ and Cu in an XRD pattern of the composite material.

2.  The copper-carbon composite material according to claim 1, having a R2 value of 0.05-0.4 : 1, preferably 0.05-0.3 : 1, wherein the R2 value is the area ratio between peaks for $Cu^+$ and $Cu^0$ obtained by peak differentiating and imitating based on the XPS spectrum and auger electron spectroscopy (XAES) of the composite material.

3.  The copper-carbon composite material according to claim 1, wherein the copper-carbon composite material shows a R1 value of 0.4-2 : 1, preferably 0.5-1.5 : 1, and a R2 value of 0.05-0.4 : 1, preferably 0.05-0.3 : 1, when measured after being kept at 25 °C for 48 hours under an air atmosphere.

4.  The copper-carbon composite material according to claim 1, wherein at least a part (preferably substantially all) of the active component is embedded into the graphite microcrystallite layers and amorphous defects of the porous carbonaceous material, and/or the copper dispersity is 5% to 20%, preferably 10% to 20%.

5.  The copper-carbon composite material according to claim 1, wherein the active component has a grain size of 3 nm to 20 nm, preferably 5 nm to 15 nm.

6.  The copper-carbon composite material according to claim 1, wherein the porous carbonaceous material is a solid carbon-based material having a carbon content of more than 80 wt%, preferably derived from at least one of petroleum coke, needle coke, pitch, biomass char and coal, more preferably derived from petroleum coke.

7.  The copper-carbon composite material according to claim 1, wherein the pore volume of pores having a pore diameter of 0.8 nm to 2nm accounts for 30% to 50%, preferably 30% to 45%, of the total pore volume.

8.  The copper-carbon composite material according to claim 1, having a specific surface area of 100-600 $m^2$/g, preferably 150-500 $m^2$/g.

9.  A method for producing a copper-carbon composite material, comprising the steps of:

    (1) contacting a carbonaceous material, a copper-containing compound and an organic solvent under heat treatment conditions to obtain a pre-composite material (referred to as material A);
    (2) contacting the material A with an alkali activator to conduct an activation reaction to obtain the copper-carbon composite material,

    wherein the carbonaceous material is a solid carbon-based material with a carbon content of more than 80wt% and is at least one selected from the group consisting of graphite precursors and activated carbon precursors.

10. The method for producing a copper-carbon composite material according to claim 9, further comprising washing and drying after completion of the activation reaction to obtain the copper-carbon composite material.

11. The method for producing a copper-carbon composite material according to claim 10, wherein the drying temperature is 50 °C to 250 °C, preferably 60 °C to 150 °C.

12. The method for producing a copper-carbon composite material according to claim 9, wherein the copper-containing compound is one or more selected from the group consisting of inorganic copper salts, organic copper salts, copper-containing oxides, and copper-containing hydroxides, specifically one or more selected from the group consisting of copper nitrate, basic copper carbonate, copper sulfate, copper chloride, copper formate, copper acetate, copper hydroxide, and copper oxide, and preferably one or more selected from the group consisting of copper chloride, copper nitrate, basic copper carbonate, copper sulfate, copper formate, and copper acetate.

13. The method for producing a copper-carbon composite material according to claim 9, wherein the organic solvent is one or more selected from the group consisting of pyrrolidone derivatives, N,N-dimethylformamide, methanol, ethanol, tetrahydrofuran, and carbon disulfide, preferably pyrrolidone derivatives, more preferably at least one selected from the group consisting of N-methyl pyrrolidone and N-ethyl pyrrolidone, and particularly preferably N-methyl pyrrolidone.

14. The method for producing a copper-carbon composite material according to claim 9, wherein the carbonaceous material is at least one selected from the group consisting of petroleum coke, needle coke, pitch, biomass char and coal, more preferably petroleum coke.

15. The method for producing a copper-carbon composite material according to claim 9, wherein in step (1), the carbonaceous material, the copper-containing compound and the organic solvent are uniformly mixed and subjected to the heat treatment, followed by drying to obtain the material A.

16. The method for producing a copper-carbon composite material according to claim 15, wherein the drying conditions include: a drying temperature of 60-350 °C, preferably 80-300 °C; and a drying time of 0.5-24h, preferably 1-18 h.

17. The method for producing a copper-carbon composite material according to claim 9, wherein the operating conditions of the heat treatment include: a treatment temperature of 60 °C to 400 °C, preferably 80 °C to 350 °C, a treatment time of 0.1 to 24 hours, preferably 1 to 16 hours, and a treatment pressure (gauge pressure) of 0 MPa to 15 MPa, preferably 1 MPa to 10 MPa, and more preferably 2 MPa to 8 MPa.

18. The method for producing a copper-carbon composite material according to claim 9, wherein the heat treatment is carried out without introduction or addition of liquid or vaporous water.

19. The method for producing a copper-carbon composite material according to claim 9, wherein the mass ratio of the carbonaceous material, the copper-containing compound, and the organic solvent is 1 : 0.01-1.2 : 1-100, preferably 1 : 0.1-0.8 : 10-50, wherein the amount of the copper-containing compound is calculated based on the mass of the Cu element.

20. The method for producing a copper-carbon composite material according to claim 9, wherein the alkali activator is one or more selected from the group consisting of potassium hydroxide, potassium carbonate, potassium bicarbonate, sodium hydroxide, and calcium hydroxide, and preferably potassium hydroxide.

21. The method for producing a copper-carbon composite material according to claim 9, wherein the mass ratio of the material A to the alkali activator is 1 : 0.2-12, preferably 1 : 0.5-6.

22. The method for producing a copper-carbon composite material according to claim 9, wherein the operating conditions for the activation reaction include: an alkali activator being in a molten state, an atmosphere of one or more of nitrogen and/or inert atmosphere, preferably nitrogen, an activation temperature of 300-600 °C, preferably 400-550 °C, and an activation time of 0.1-10 h, preferably 0.5-8 h.

23. The method for producing a copper-carbon composite material according to claim 9, wherein the activation reaction is carried out without introduction or addition of liquid or vaporous water.

24. Use of the copper-carbon composite material according to claim 1 or the copper-carbon composite material obtained by the method according to claim 9 in the production of methanol by hydrogenation of carbon dioxide.

25. Use of the copper-carbon composite material according to claim 1 or the copper-carbon composite material obtained by the method according to claim 9 in the adsorptive separation of CO.

26. Use of the copper-carbon composite material according to claim 1 or the copper-carbon composite material obtained by the method according to claim 9 in the production of lower hydrocarbons by hydrogenation of carbon dioxide.

27. Use of the copper-carbon composite material according to claim 1 or the copper-carbon composite material obtained by the method according to claim 9 in the photocatalytic conversion of carbon dioxide.

28. Use of the copper-carbon composite material according to claim 1 or the copper-carbon composite material obtained by the method according to claim 9 in the electrocatalytic conversion of carbon dioxide.

**Fig. 1**

**Fig. 2**

Pore Width(nm)

**Fig. 3**

**INTERNATIONAL SEARCH REPORT**

| International application No. |
|---|
| **PCT/CN2023/098776** |

**A.    CLASSIFICATION OF SUBJECT MATTER**

B01J21/18(2006.01)i；  B01J23/72(2006.01)i；  B01J37/00 (2006.01)i

According to International Patent Classification (IPC) or to both national classification and IPC

**B.    FIELDS SEARCHED**

Minimum documentation searched (classification system followed by classification symbols)

IPC:B01J21/-、 B01J23/-、 B01J37/-

Documentation searched other than minimum documentation to the extent that such documents are included in the fields searched

Electronic data base consulted during the international search (name of data base and, where practicable, search terms used)

CNABS, CNTXT, ENTXT, CNKI, VEN, Web of Science: Cu2O, Cu, 铜, 氧化亚铜, 碳, 二氧化碳, CO2, copper, cuprous oxide, carbon, carbon dioxide

**C.    DOCUMENTS CONSIDERED TO BE RELEVANT**

| Category* | Citation of document, with indication, where appropriate, of the relevant passages | Relevant to claim No. |
|---|---|---|
| X | CN 106540694 A (LIAONING SHIHUA UNIVERSITY) 29 March 2017 (2017-03-29) description, paragraphs 4-24 | 1-8 |
| X | CN 109701535 A (NORTH UNIVERSITY OF CHINA) 03 May 2019 (2019-05-03) description, paragraphs 6-16 | 1-8 |
| A | CN 111377443 A (CHINA PETROLEUM & CHEMICAL CORP. et al.) 07 July 2020 (2020-07-07) entire document | 1-28 |
| A | KR 20160116112 A (KOREA INSTITUTE OF INDUSTRIAL TECHNOLOGY) 07 October 2016 (2016-10-07) entire document | 1-28 |
| A | US 2013256123 A1 (RAHMAN SALEEM UR et al.) 03 October 2013 (2013-10-03) entire document | 1-28 |
| A | US 2021253496 A1 (BP P.L.C.) 19 August 2021 (2021-08-19) entire document | 1-28 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **11 September 2023** | **23 September 2023** |

| Name and mailing address of the ISA/CN | Authorized officer |
|---|---|
| **China National Intellectual Property Administration (ISA/ CN) China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

| Patent document cited in search report | | | Publication date (day/month/year) | Patent family member(s) | | | Publication date (day/month/year) |
|---|---|---|---|---|---|---|---|
| CN | 106540694 | A | 29 March 2017 | None | | | |
| CN | 109701535 | A | 03 May 2019 | None | | | |
| CN | 111377443 | A | 07 July 2020 | None | | | |
| KR | 20160116112 | A | 07 October 2016 | KR | 101761752 | B1 | 27 July 2017 |
| US | 2013256123 | A1 | 03 October 2013 | US | 2014336037 | A1 | 13 November 2014 |
| | | | | US | 9109293 | B2 | 18 August 2015 |
| US | 2021253496 | A1 | 19 August 2021 | JP | 2021525772 | A | 27 September 2021 |
| | | | | EP | 3802470 | A1 | 14 April 2021 |
| | | | | US | 11613505 | B2 | 28 March 2023 |
| | | | | WO | 2019233961 | A1 | 12 December 2019 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 112121805 A

- CN 111215084 A